# EUROPÄISCHE PATENTSCHRIFT

(11) **EP 1 706 071 B1**
(45) Veröffentlichungstag und Bekanntmachung des Hinweises auf die Patenterteilung: **02.09.2009**
(21) Anmeldenummer: 05715196.1
(22) Anmeldetag: 20.01.2005
(51) Int. Cl.: A61F 2/18

(54) **GEHÖRKNÖCHELCHENPROTHESE**
AUDITORY OSSICLES PROSTHESIS
PROTHÈSE D'OSSELETS DE L'OREILLE

(30) Priorität: 23.01.2004 DE 202004001008 U
(43) Veröffentlichungstag der Anmeldung: 04.10.2006
(73) Patentinhaber: Heinz Kurz GmbH Medizintechnik, 72144 Dusslingen (DE)
(72) Erfinder: STEINHARDT, Uwe, 72145 Hirrlingen (DE); AWENGEN, Daniel, F., CH-4102 Binningen (CH)
(74) Vertreter: Kohler Schmid Möbus
(86) Internationale Anmeldenummer: PCT/EP2005/000528
(87) Internationale Veröffentlichungsnummer: WO 2005/070327

(56) Entgegenhaltungen:
- WO-A-90/07915
- WO-A-90/11737
- WO-A-95/01710
- WO-A-99/15111
- DE-U1- 20 310 609
- US-A- 4 655 776
- MEISTER H; WALGER M; MICKENHAGEN A; VON WEDEL H; STENNERT E: "Standardized measurements of the sound transmission of middle ear implants using a mechanical middle ear model" EUROPEAN ARCHIVES OF OTO-RHINO-LARYNGOLOGY, Bd. 256, Nr. 3, 31. März 1999 (1999-03-31), Seiten 122-127, XP002334807

## Beschreibung

Die Erfindung betrifft eine Gehörknöchelchenprothese, die mindestens ein Glied der menschlichen Gehörknöchelchenkette ersetzen oder überbrücken kann, wobei die Gehörknöchelchenprothese aus einem elastischen Material oder aus einem mindestens eine Gelenkverbindung aufweisenden Material gefertigt ist, und wobei Mittel zur Frequenzanpassung (=Tuning) für die Schallleitung im Mittelohr vorgesehen sind.

Eine derartige Gehörknöchelchenprothese ist aus der WO 90/11737 A bekannt, die als nächstliegender Stand der Technik angesehen wird.

Gehörknöchelchenprothesen werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise am Ambossfortsatz der menschlichen Gehörknöchelchenkette befestigt und das andere Ende der Gehörknöchelchenprothese beispielsweise am Steigbügel der menschlichen Gehörknöchelchenkette befestigt oder direkt ins Innenohr getaucht wird.

Da die anatomischen Gegebenheiten des Ohrs, wie beispielsweise die Lage, die Form und die Größe des Steigbügels, des Ambosses, des Hammers und des Trommelfells variieren, ist es vorteilhaft, wenn Gehörknöchelchenprothesen nicht starr ausgebildet sind, sondern eine gewisse Flexibilität oder Variabilität aufweisen. Um diese Flexibilität/Variabilität zu erreichen sind verschiedene Befestigungs- und Ankopplungsvorrichtungen für Gehörknöchelchen, die elastische Teile und/oder Gelenke aufweisen, bekannt. Vielfach wird mit den bekannten Gehörknöchelchenprothesen die Schallleitung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Aufgabe der Erfindung ist es demgegenüber, eine Gehörknöchelchenprothese der eingangs beschriebenen Art so zu modifizieren, dass damit die Schallleitung zwischen dem Mittelohrbereich und dem Innenohr des menschlichen Gehörganges erheblich verbessert wird, wobei insbesondere auch eine optimale Anpassung an die individuell unterschiedlichen Verhältnisse und eine maßgeschneiderte Lösung der Probleme und Defekte beim jeweiligen Patienten ermöglicht werden soll.

Erfindungsgemäß wird diese Aufgabe auf ebenso einfach anmutende wie wirkungsvolle Art und Weise dadurch gelöst, dass die Mittel zur Frequenzanpassung Hebelelemente zur Veränderung der Hebelverhältnisse in der Gehörknöchelchenkette umfassen, und dass die Prothese mindestens eine Kugelgelenkverbindung aufweist.

Um die erfindungsgemäße Gehörknöchelchenprothese individuell besonders gut an die komplexen und in jedem Einzelfall unterschiedlich ausgestalteten Strukturen im Mittelohr anpassen zu können, beispielsweise zur Vermeidung unzulässiger mechanischer Kontakte zu den zahlreichen im Mittelohr verlaufenden Nervenbahnen, umfassen bei der erfindungsgemäßen Prothese die Mittel zur Frequenzanpassung Hebelelemente, deren effektive Längen bzw. Hebelverhältnisse in Abhängigkeit von einem vorgegebenen Frequenzgang veränderbar sind.

Die erfindungsgemäße Gehörknöchelchenprothese hat gegenüber bekannten Vorrichtungen den wesentlichen Vorteil, dass damit gezielt eine Frequenz abhängige Hörverbesserung erreicht werden kann. Beispielsweise Hörprobleme in Form von sogenannten Löchern im gehörten Frequenzband, die sich durch einen drastischen Abfall der Hörleistung des Patienten in bestimmten, oftmals relativ eng begrenzten Bereichen des mittels eines Audiogramms dargestellten individuellen Hörspektrums manifestieren, können gezielt durch ein entsprechendes Tuning behoben oder zumindest wesentlich gelindert werden. Das dadurch erzielbare Hörresultat beim Patienten kann auf diese Weise ganz erheblich verbessert werden. Außerdem eröffnet die erfindungsgemäße Idee eine große Vielzahl von unterschiedlichen Eingriffs- und Ansatzmöglichkeiten zur individuellen Therapie von Hörproblemen im Mittelohrbereich, die weit über die Behandlungsmöglichkeiten beim Einsatz bekannter Gehörknöchelchenprothesen hinausgehen.

Bei einer bevorzugten Klasse von Ausführungsformen der Erfindung ist der Übergang vom Ambossfortsatz zum Steigbügel weitestgehend den anatomischen Verhältnissen der natürlichen Gehörknöchelchenkette nachgebildet. Insbesondere zeichnen sich diese Ausführungsformen dadurch aus, die Prothese einerseits am Ambossfortsatz und andererseits am Steigbügel befestigt ist oder direkt ins Innenohr getaucht wird, von ihrer Anlenkung am Ambossfortsatz ausgehend den Verlauf des natürlichen Ambossfortsatzes bis zu seinem Ende oder darüber hinaus weitgehend nachbildet und im Bereich der Höhe des natürlichen Endes des Ambossfortsatzes abgewinkelt zum anderen Endpunkt der Gehörknöchelchenprothese am Steigbügel oder am/im Innenohr verläuft.

Die Anbindung der Prothese am Ambossfortsatz kann ca. 1 mm hinter dem distalen Ende des Fortsatzes erfolgen und über die Ausrichtung der Prothese in Richtung des Verlaufs des natürlichen Fortsatzes können Hebelverhältnisse erreicht bzw. simuliert werden, die weitestgehend den natürlichen Verhältnissen entsprechen. Die Prothese ist in ihrem Verlauf gelenkig bzw. elastisch abgewinkelt, damit, wie bei der natürlichen Gehörknöchelchenkette, die Schallleitung den Raumverhältnissen im Mittelohr angepasst erfolgen kann. Die Hebelverhältnisse sind bei der erfindungsgemäßen Prothese gegenüber den aus dem Stand der Technik bekannten Prothesen erheblich verbessert, so dass mit der neuen Prothese ein wesentlich verbesserter Hörkomfort erreicht wird.

Die erfindungsgemäße Prothese selbst kann aus gewebe- und knochenverträglichen Kunststoffen, Faserverbundwerkstoffen oder Metallen hergestellt sein, die den abgewinkelten Verlauf der erfindungsgemäßen Prothese in ihrer Beweglichkeit unterstützen bzw. gewährleisten.

In einer bevorzugten Ausgestaltung der Erfindung ist die Prothese über einen ersten Clip am Ambossfortsatz befestigt, an dem ein erster Stab ausgebildet ist, der in einem als Kugel ausgebildeten Ende endet, das in einem U-förmigen Pfannenteil gelagert ist, das in einen zweiten Stab übergeht, der als Kolben oder in einem weiteren Clip endet.
Mit dieser gegenständlichen Ausgestaltung der erfindungsgemäßen Gehörknöchelchenprothese wird eine hohe Beweglichkeit erreicht, die die Schallleitung im Mittelohr verbessert unterstützt. Mittels des Kugelgelenks wird eine sehr hohe Beweglichkeit der erfindungsgemäßen Prothese erreicht und dies in einem Verlauf, der der menschlichen Gehörknöchelchenkette nachempfunden ist.

Wird die erfindungsgemäße Prothese über Clips bzw. über einen Kolben am Ambossfortsatz bzw. am Steigbügel befestigt oder über einen Kolben direkt in das Innenohr getaucht, so wird die Flexibilität oder Beweglichkeit der erfindungsgemäßen Prothese nicht behindert.

Eine besonders bevorzugte Ausführungsform eines Gelenks im abgewinkelten Bereich der Prothese wird über eine Kugel und ein U-förmiges Pfannenteil gebildet, in dem die Kugel in den Seitenwänden des Pfannenteils in Öffnungen des Pfannenteils gelagert ist. Über eine derartige konstruktive Ausgestaltung kann sich die Kugel im U-förmig ausgebildeten Pfannenteil uneingeschränkt in alle Richtungen bewegen und Schallleitungslösungen im relativ engen Mittelohrraum bestmöglich gewährleisten.

In weiterer Ausgestaltung der Erfindung sind der erste und/oder weitere Clip aus zwei V- oder U-förmig angeordneten Federzungen gebildet. Der Halt eines Clips wird über eine derartige Ausgestaltung verbessert. Sind die Kontaktstellen der Clips noch an Stellen aufgeraut, an denen sie am Ambossfortsatz bzw. am Steigbügel aufliegen, so ist eine gesicherte, dauerhafte Befestigung der erfindungsgemäßen Gehörknöchelchenprothese durch erhöhte Friktion und einer daraus folgenden innigen Verbindung gewährleistet.

Zur erleichterten Platzierung der erfindungsgemäßen Gehörknöchelchenprothese ist an dem ersten und/oder weiteren Clip jeweils ein Haltegriff ausgebildet. Diese Ausgestaltung erleichtert die Anbringung der erfindungsgemäßen Prothese im Mittelohr und verringert das Risiko einer Beschädigung der Prothese während der operativen Implantation.

Die erfindungsgemäße Gehörknöchelchenprothese kann an einem Ende als Kolben ausgebildet sein, der direkt in das Innenohr eingetaucht wird. Bei dieser Ausführungsform ist ein weiterer Clip nicht notwendig. Insbesondere kann die Prothese mittels Eröffnung der menschlichen Hörschnecke (=Cochleotomie) einenends direkt an das Innenohr angekoppelt werden. Damit wird ein besonders langer Hebelarm erzielt, so dass die Verstärkungswirkung aufgrund des Hebelweges und der entsprechenden großen Auslenkung optimal ist.

Bei einer weiteren Klasse von Ausführungsformen der Erfindung kann die Prothese einerseits am Hammer (=Malleus), und andererseits am Amboss oder am Steigbügel befestigt sein oder wiederum, wie schon oben beschrieben, direkt ins Innenohr eingetaucht werden. Auf diese Weise wird dem Therapeuten eine große Variabilität in der Wahl seiner auf den individuellen Einzelfall optimal angepassten Therapiemethode eröffnet. So können sämtliche möglichen Defekte und Probleme im Mittelohrbereich des Patienten maßgeschneidert behandelt werden.

Der Hebelweg und die hierdurch erzielbare Auslenkung wird umso größer sein, je näher die Prothese am Endpunkt (=Umbo) des Hammers angeordnet ist. Daher können Weiterbildungen der obigen Ausführungsformen vorteilhaft sein, bei denen die Prothese am Umbo oder direkt daneben angeordnet ist. Hierdurch lässt sich eine besonders hohe Verstärkungswirkung für das durch das Mittelohr an das Innenohr weiter zu leitende Schallsignal erzielen.

Die erfindungsgemäße Gehörknöchelchenprothese selbst ist aus einem biokompatiblen Material bzw. Materialverbund hergestellt, welches toxische Reaktionen in der Mittelohrumgebung ausschließt und Reizungen aufgrund des Implantatwerkstoffs so weit wie möglich verhindert.

Bei Ausführungsformen der Erfindung kann die Prothese oder Teile davon aus biokompatiblen Kunststoffen oder Kunststoffteilen, insbesondere Silikon, oder Faserverbundwerkstoffen hergestellt sein, was optimale Behandlungsmöglichkeiten für den Therapeuten eröffnet.

Bei weiteren Ausführungsformen kann die Prothese auch Metallkomponenten aus Titan und/oder Gold und/oder Tantal und/oder einer Legierungen dieser Metalle enthalten, wobei hierbei die Duktilität und gute Sterilisierbarkeit der Materialien ausgenutzt werden kann. Außerdem kann die Prothese bei Verwendung von Metallkomponenten leicht in tomographischen Aufnahmen mittels Magnetresonanz sichtbar gemacht werden.

Vorteilhaft kann es auch sein, die Prothese oder Teile davon aus einem Material mit Formgedächtnis (=memory effect), insbesondere aus Nitinol herzustellen. Hiermit lässt sich insbesondere eine berührungslose Ankopplung der Prothese bei der Implantation im Zielgebiet des Mittelohres erreichen.

Die Prothese selbst kann vollständig oder auch nur teilweise aus einem oder mehreren der oben genannten Metalle gefertigt sein.

Bei bevorzugten Ausführungsformen der Erfindung können die Mittel zur Frequenzanpassung eine Einrichtung zum Verändern der Anlenkungsstelle der Prothese am Hammer und/oder am Ambossfortsatz und/oder am Steigbügel und/oder am Innenohr in Abhängigkeit von einem vorgegebenen Frequenzgang umfassen. Hierdurch wird dem behandelnden Therapeuten eine hohe Variabilität bei der Auswahl der optimalen Prothese in Abhängigkeit von der vorgefundenen Situation im Mittelohr des Patienten eröffnet.

Bei vorteilhaften Ausführungsformen umfassen die Hebelelemente einen Hebelabschnitt, der das natürliche Ende des Ambossfortsatzes verlängert. Damit kann z.B. bei angeborenen oder krankhaften Verkürzungen des Ambossfortsatzes ein normaler bzw. der ursprüngliche Aufbau und Verlauf der Gehörknöchelchenkette hergestellt bzw. rekonstruiert werden.

Eine einfache und in ihrer Wirkung (relativ) leicht vorher bestimmbare mechanische Beeinflussung der akustischen Reaktion einer zu behandelnden Gehörknöchelchenkette kann dadurch erreicht werden, dass die Massenverteilung der einzelnen Teile der Prothese in Abhängigkeit von einem gewünschten, vorgebbaren Frequenzgang der Schallleitung im Mittelohr berechnet wird.

Besonders einfach zu realisieren ist eine Ausführungsform der Erfindung, bei der mindestens eine zusätzliche Masse in Abhängigkeit von einem gewünschten, vorgebbaren Frequenzgang der Schallleitung im Mittelohr an einem Teil der Gehörknöchelchenkette befestigt wird. Dies kann ggf. auch im Sinne eines Fein-Tunings mit der oben beschriebenen Maßnahme kombiniert werden. So lässt sich die Impedanz bzw. das Spektrum der Resonanzfrequenzen der Kette individuell und in genau vorgebbaren Grenzen ohne großen Aufwand in eine gewünschte Richtung verschieben.

Bei intraoperativ leicht zu handhabenden Weiterbildungen der obigen Ausführungsformen wird die zusätzliche Masse mittels eines Clips an einem Teil der Gehörknöchelchenkette befestigt.

Schließlich können Ausführungsformen der erfindungsgemäßen Gehörknöchelchenprothese auch mit einem aktiven Vibrationsteil eines aktiven, implantierbaren Hörgeräts verbunden sein, um eine Versorgung von Patienten mit Innenohr- und Mittelohr-Schwerhörigkeit zu erleichtern.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der erfindungsgemäßen Gehörknöchelchenprothese dargestellt, welche in der nachfolgenden Beschreibung näher erläutert werden. Die in der Zeichnung dargestellten Ausführungsformen sind beispielhaft zu verstehen und zeigen den erfindungsgemäßen Gegenstand nicht notwendig maßstäblich.

Es zeigen:
- **Fig. 1**: eine erfindungsgemäße Gehörknöchelchenprothese in räumlicher Darstellung mit einer Abwinklung, die als Gelenk ausgebildet ist;
- **Fig. 2**: eine Gehörknöchelchenprothese gemäß Fig. 1 aus einem anderen Sichtwinkel;
- **Fig. 3**: einen Ausschnitt einer erfindungsgemäßen Gehörknöchelchenprothese gemäß Fig. 1 und 2, wie sie am Ambossfortsatz angelenkt bzw. befestigt ist;
- **Fig. 4a**: eine Ausführungsform der erfindungsgemäßen Gehörknöchelchenprothese mit Anlenkung am Ambossfortsatz einerseits, Gelenk und Durchtritt durch die Steigbügelfußplatte andererseits;
- **Fig. 4b**: wie Fig. 4a, aber mit verlängertem Hebel zwischen Ambossfortsatz und Gelenk;
- **Fig. 4c**: wie Fig. 4b, aber mit zusätzlicher Masse an der Prothese;
- **Fig. 5**: eine Ausführungsform der erfindungsgemäßen Gehörknöchelchenprothese mit Anlenkung am Hammergriff einerseits, Gelenk und Durchtritt durch die Steigbügelfußplatte andererseits; und
- **Fig. 6**: eine Ausführungsform der erfindungsgemäßen Gehörknöchelchenprothese mit Anlenkung am Hammergriff einerseits, mehrgliedrigem Gelenk und Befestigung am Steigbügel andererseits.

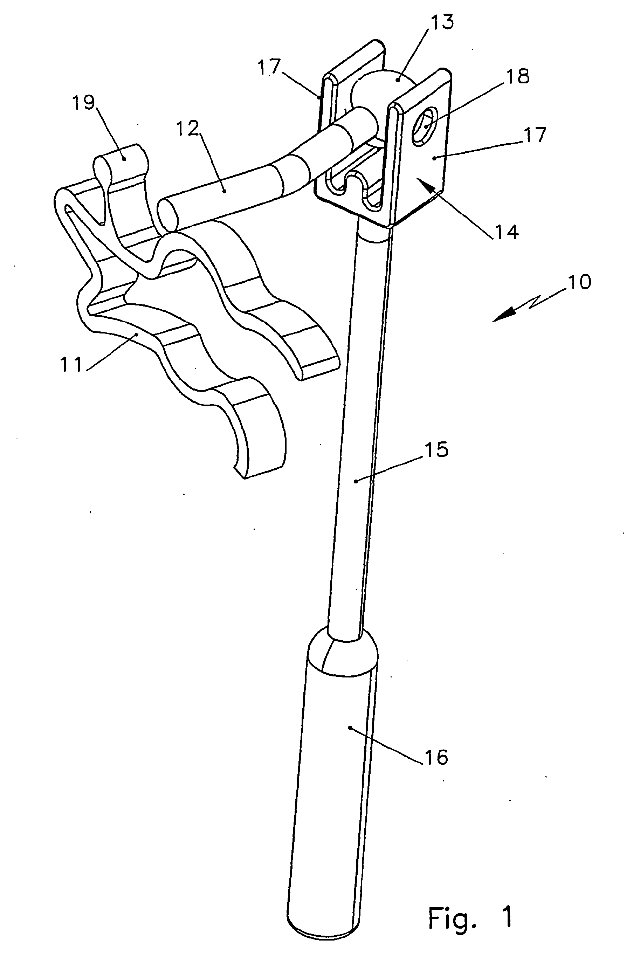

**Fig. 1** zeigt in perspektivischer Darstellung eine erfindungsgemäße Gehörknöchelchenprothese **10,** die einen ersten Clip **11** aufweist, an dem ein erster Stab **12** befestigt ist. Der erste Stab 12 verläuft leicht angewinkelt, er muss nicht geradlinig verlaufen. Der Stab 12 ist materialschlüssig mit dem ersten Clip 11 verbunden, der als V-förmiger Clip mit hoher Flexibilität ausgebildet ist. Der erste Clip 11 wie auch der erste Stab 12 können aus einer Titanlegierung hergestellt sein.

Der erste Stab 12 endet, dem ersten Clip 11 gegenüberliegend, in einer Kugel **13,** die in einem U-förmigen Pfannenteil **14** gelagert ist. An das U-förmige Pfannenteil 14 schließt sich ein zweiter Stab **15** an, der in einen Kolben **16** übergeht. Anstatt des Kolbens 16 kann ein weiterer Clip am zweiten Stab 15 ausgebildet sein. Über den ersten Clip 11 bzw. den Kolben 16 oder den alternativ am Ende des zweiten Stabs 15 ausgebildeten weiteren Clip ist die erfindungsgemäße Gehörknöchelchenprothese 10 im Mittelohr einerseits am Ambossfortsatz und andererseits am Steigbügel bzw. am/im Innenohr gehalten.

Das U-förmige Pfannenteil 14 weist Seitenwände **17** auf, in denen jeweils Öffnungen **18** ausgebildet sind. In diesen Öffnungen 18 ist die Kugel 13 gelenkig gelagert gehalten, sodass eine hohe Beweglichkeit der Gehörknöchelchenprothese 10 zwischen dem ersten Clip 11 und dem Kolben 16 gegeben ist. Am ersten Clip 11 ist ein Haltegriff **19** ausgebildet, über den die Platzierung des ersten Clips 11 am Ambossfortsatz erleichtert wird.

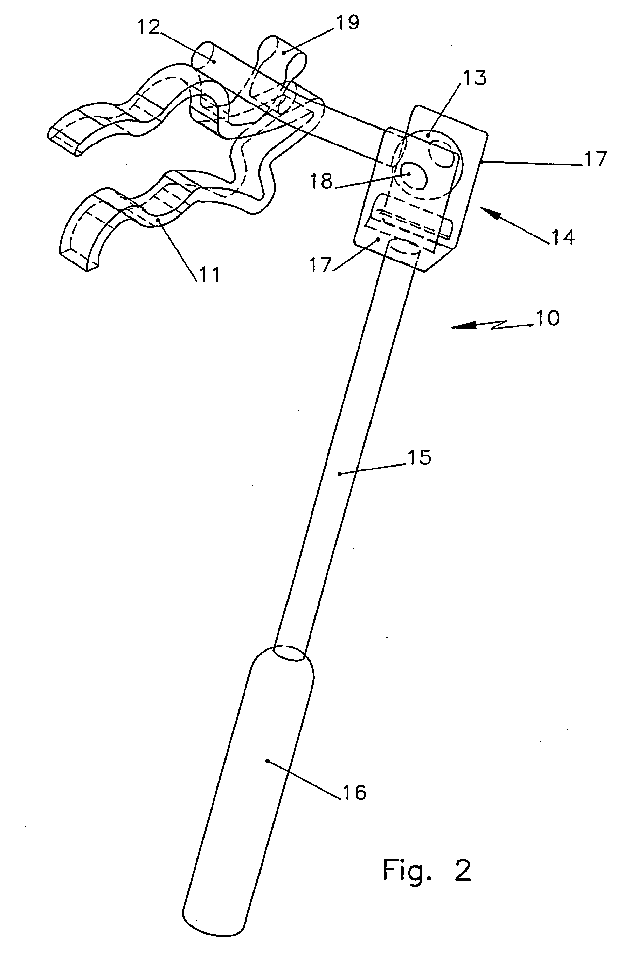

**Fig. 2** zeigt die erfindungsgemäße Gehörknöchelchenprothese 10 aus Fig. 1 in einer weiteren perspektivischen Darstellung aus einem anderen Sichtwinkel, wobei dieselben gegenständlichen Merkmale mit denselben Bezugszeichen versehen sind. Die Anlenkung des ersten Stabs 12 an den zweiten Stab 15 über das U-förmige Pfannenteil 14 ist deutlich zu erkennen und auch die hohe Beweglichkeit der Kugel 13, gelagert in den Öffnungen 18 der Seitenwände 17 des U-förmigen Pfannenteils 14, ist deutlich gezeigt. Ist die erfindungsgemäße Gehörknöchelchenprothese 10 im Mittelohr platziert, so besteht über das Kugelgelenk eine hohe Beweglichkeit zwischen dem ersten Clip 11 und dem Kolben 16.

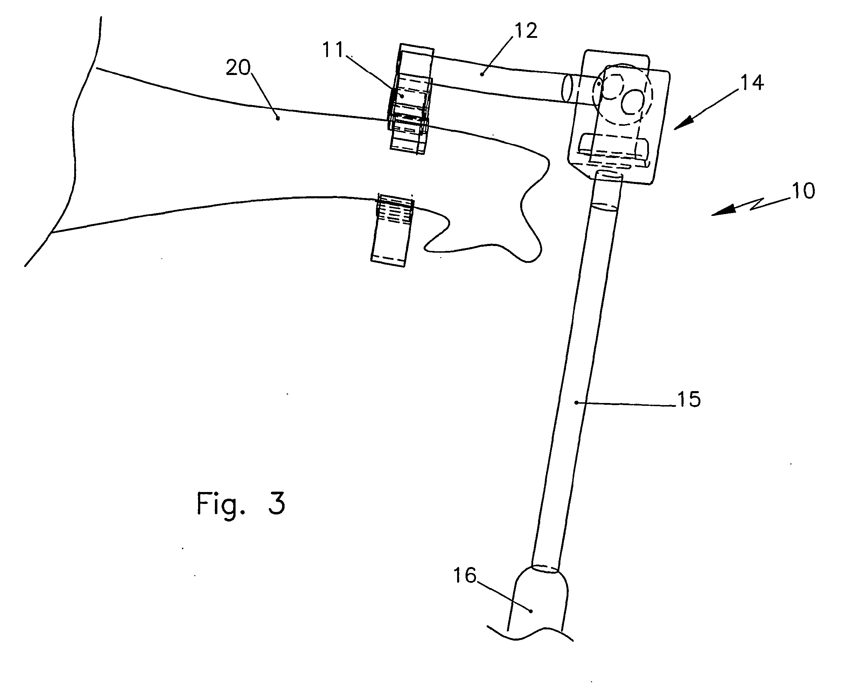

**Fig. 3** zeigt einen Ausschnitt sowohl eines Ambossfortsatzes **20** wie auch der in den Fig. 1 und 2 gezeigten Gehörknöchelchenprothese 10, wie sie am Ambossfortsatz 20 über den ersten Clip 11 befestigt ist. Die Gehörknöchelchenprothese 10 ragt über den natürlichen Ambossfortsatz 20 hinaus, indem der erste Stab 12 gelenkig verbunden in den zweiten Stab 15 übergeht, der in dem noch teilweise gezeigten Kolben 16 endet.

Mit der in den Figuren 1 bis 3 gezeigten Gehörknöchelchenprothese 10 wird eine erste Klasse von erfindungsgemäßen Prothesen gezeigt, über die die Hebelverhältnisse bzw. das Tuning des Mittelohrs für Schallleitungen erheblich verbessert werden kann. Weitere Ausführungsformen zeigen die folgenden Figuren:

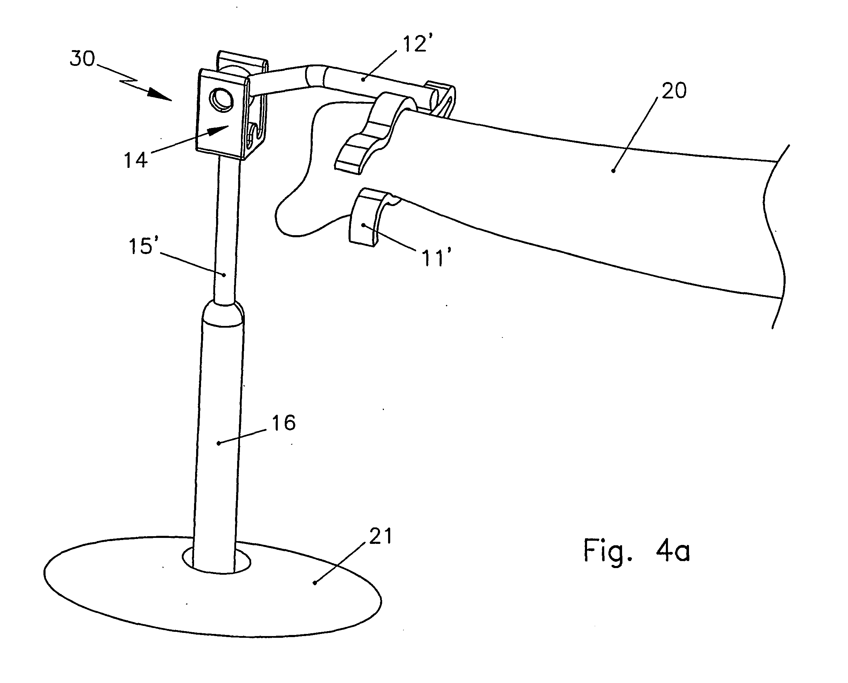

**Fig. 4a** stellt schematisch eine Gehörknöchelchenprothese **30** dar, die einerseits wieder über einen ersten Clip **11'** am Ambossfortsatz 20 befestigt ist. Am ersten Clip 11' greift wiederum ein erster Stab **12'** an, der über ein U-förmiges Pfannenteil 14 gelenkig mit dem zweiten Stab **15'** verbunden ist, welcher am anderen Ende wieder in den Kolben 16 übergeht, der allerdings bei diesem Ausführungsbeispiel durch eine Öffnung in der Steigbügelfußplatte **21** in das (nicht dargestellte) Innenohr ragt.

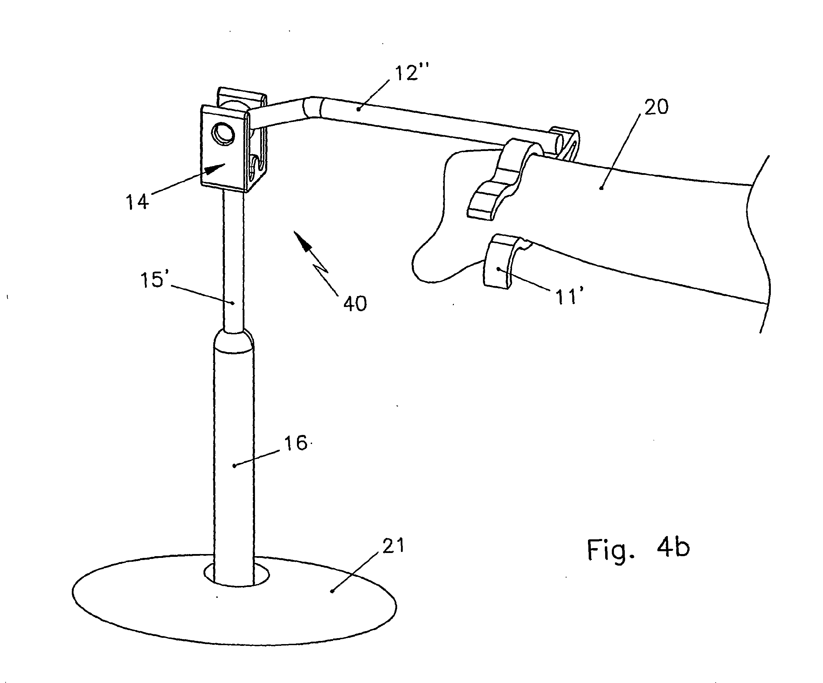

Die Ausführungsform nach **Fig. 4b** unterscheidet sich von der nach Fig. 4a darin, dass der erste Stab **12"** der Gehörknöchelchenprothese 40 gegenüber dem ersten Stab 12' der Gehörknöchelchenprothese 30 zwischen dem ersten Clip 11' und dem gelenkigen Endpunkt des ersten Stabes in der (in der Zeichnung nicht erkennbaren) Kugel des U-förmiges Pfannenteils 14 erheblich verlängert ist und damit einen wesentlich größeren Hebelweg aufweist. Je nach individueller Situation im Mittelohr des Patienten wird der Abstand zwischen erstem Clip 11,11' und der Kugel 13 in einem Bereich von 1 bis 5 mm gewählt werden. Der Abstand zwischen der Kugel 13 und der Steigbügelfußplatte 21 wird in einem Bereich zwischen 3 und 7 mm liegen.

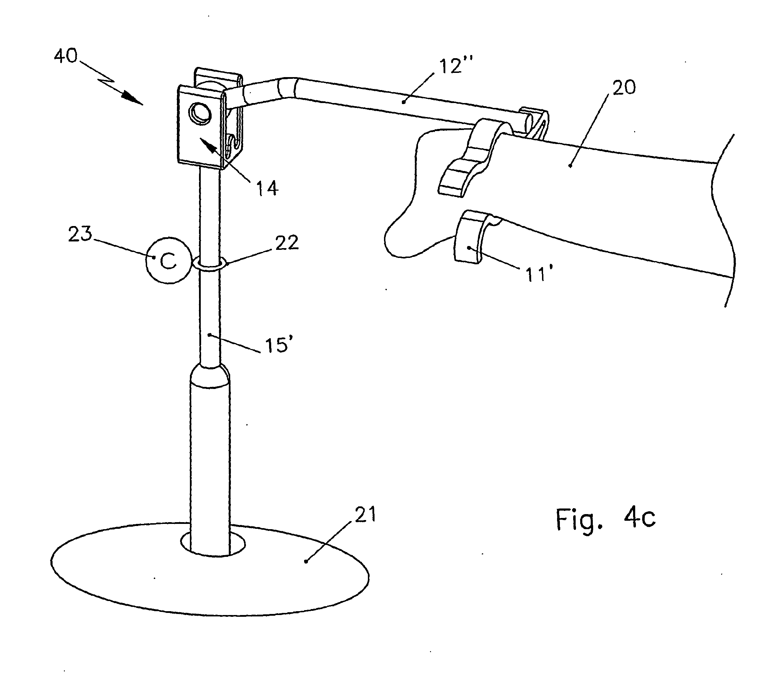

Zur weiteren Verbesserung der Hörqualität ist in der Ausführungsform nach **Fig. 4c** über einen zweiten Clip **22** eine zusätzliche Masse **23** am zweiten Stab 15' angebracht. Diese dient einem Feintuning der akustischen Eigenschaften der Gehörknöchelchenprothese 40 durch gezielte Verschiebung der Resonanzfrequenz auf einen gewünschten Wert.

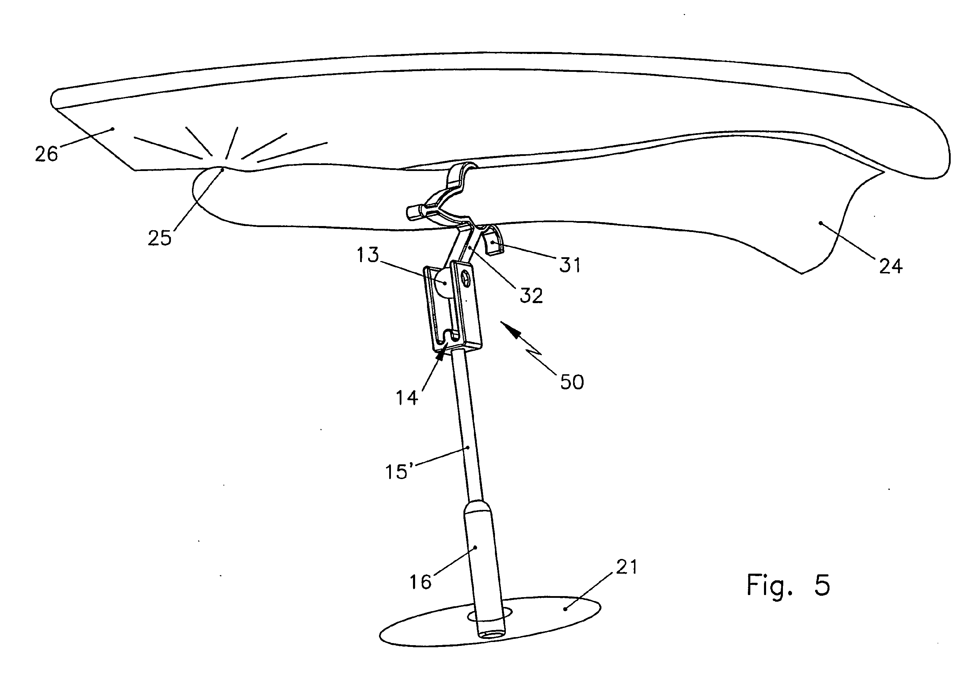

**Fig. 5** zeigt eine weitere Ausführungsform, bei welcher die Gehörknöchelchenprothese 50 über einen ersten Clip **31** am Hammergriff **24** in der Nähe des Umbo **25** befestigt ist, wo der Hammer mechanischen Kontakt mit dem Trommelfell **26** hat. Anstelle des ersten Stabes ist bei dieser Ausführungsform der Clip 31 über eine Verlängerung 32 direkt mit der Kugel 13 des U-förmigen Pfannenteils 14 verbunden, an dessen anderen Ende wieder ein zweiter Stab 15' angreift, der in den Kolben 16 übergeht, welcher durch eine Öffnung in der Steigbügelfußplatte 21 in den Innenohrraum ragt.

Der Abstand zwischen der Kugel 13 und der Steigbügelfußplatte 21 wird je nach individueller Situation des Patienten in einem Bereich von 3 bis 6 mm gewählt, der Abstand zwischen Kugel 13 und Hammergriff 24 über die Länge der Verlängerung 32 in einem Bereich von 1,5 bis 3 mm.

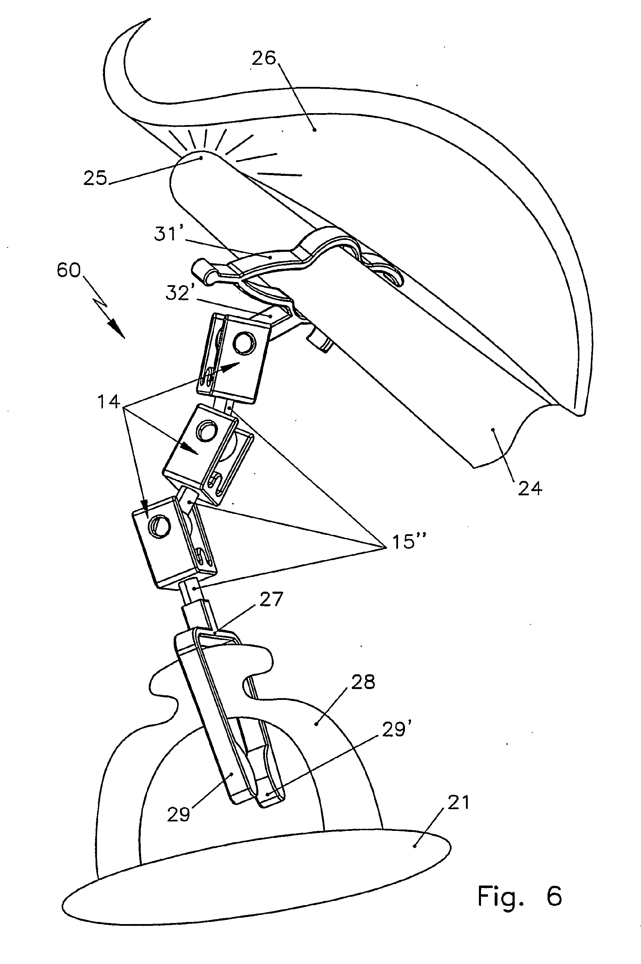

**Fig. 6** schließlich stellt eine Gehörknöchelchenprothese **60** dar, die wie die Ausführungsform nach Fig. 5 an ihrem einen Ende über einen ersten Clip **31'** am Hammergriff 24 in der Nähe des Umbo 25 befestigt ist. An ihrem anderen Ende ist die Gehörknöchelchenprothese 60 jedoch mittels eines dritten Clips **27** mit dem Steigbügel **28** verbunden. Die Länge der beiden Schenkel **29, 29'** des dritten Clips 27 wird in einem Bereich von 1 bis 3 mm, in der Regel zwischen 1,5 und 2,5 mm gewählt werden.

Um eine hohe Gelenkigkeit zu erhalten, können Ausführungsformen der Erfindung eine Vielzahl von aneinander angreifenden Gelenkstellen aufweisen. Die in Fig. 6 gezeigte Gehörknöchelchenprothese 60 umfasst beispielweise drei über verkürzte Stäbe **15"** zu einer Kette verbundene U-förmige Pfannenteile 14, von denen das erste mit der Verlängerung **32'** des ersten Clips 31' und das dritte über seinen Stab 15" mit dem dritten Clip 27 verbunden ist. Die Länge der Gelenkkette zwischen dem ersten Clip 31' und dem dritten Clip 27 wird in einem Bereich von 2 bis 6 mm gewählt werden.

Die Durchmesser der Kugeln 13 in den U-förmigen Pfannenteilen 14 werden in der Regel etwa 0,5 mm betragen.

## Patentansprüche

1. Gehörknöchelchenprothese (10; 30; 40; 50; 60), die aus einem elastischen Material oder aus einem mindestens eine Gelenkverbindung aufweisenden Material gefertigt ist, wobei Mittel zur Frequenzanpassung (=Tuning) für die Schallleitung im Mittelohr vorgesehen sind, **dadurch gekennzeichnet, dass** die Mittel zur Frequenzanpassung Hebelelemente zur Veränderung der Hebelverhältnisse in der Gehörknöchelchenkette umfassen, und dass die Prothese mindestens eine Kugelgelenkverbindung aufweist.

2. Gehörknöchelchenprothese nach Anspruch 1, **dadurch gekennzeichnet, dass** die Prothese einen ersten Clip (11; 11') aufweist, über den sie am Ambossfortsatz (20) befestigt werden kann, und dass an dem ersten Clip (11; 11') ein erster Stab (12; 12'; 12") ausgebildet ist, der in einem als Kugel (13) ausgebildeten Ende endet, das in einem U-förmigen Pfannenteil (14) gelagert ist, das in einen zweiten Stab (15; 15'; 15") übergeht, der als Kolben (16) oder in einem weiteren Clip endet.

3. Gehörknöchelchenprothese nach Anspruch 2, **dadurch gekennzeichnet, dass** der U-förmige Pfannenteil (14) in Seitenwänden (17) Öffnungen (18) aufweist, in denen die Kugel (13) gelagert ist.

4. Gehörknöchelchenprothese nach Anspruch 2 oder 3, **dadurch gekennzeichnet, dass** der erste und/oder weitere Clip (11; 11') aus zwei V- oder U-förmig angeordneten Federzungen gebildet ist.

5. Gehörknöchelchenprothese nach einem der Ansprüche 2 bis 4, **dadurch gekennzeichnet, dass** der erste und/oder weitere Clip (11; 11') an seinen Kontaktstellen zum Ambossfortsatz (20) und/oder zum Steigbügel aufgeraut ist.

6. Gehörknöchelchenprothese nach einem der Ansprüche 2 bis 5, **dadurch gekennzeichnet, dass** der erste und/oder weitere Clip (11) einen Haltegriff (19) aufweist.

7. Gehörknöchelchenprothese nach einem der vorhergehenden Ansprüche, **dadurch gekennzeichnet, dass** die Gehörknöchelchenprothese (30; 40; 50) so ausgebildet ist, dass sie mittels Eröffnung der menschlichen Hörschnecke (=Cochleotomie) einenends direkt an das Innenohr angekoppelt werden kann, insbesondere über einen Kolben (16).

8. Gehörknöchelchenprothese nach einem der vorhergehenden Ansprüche, **dadurch gekennzeichnet, dass** die Prothese oder Teile davon aus biokompatiblen Kunststoffen, insbesondere Silikon, oder Faserverbundwerkstoffen hergestellt ist.

9. Gehörknöchelchenprothese nach einem der vorhergehenden Ansprüche, **dadurch gekennzeichnet, dass** die Prothese oder Teile davon aus Titan und/oder aus Gold und/oder aus Tantal und/oder aus einer Legierung der genannten Metalle hergestellt ist.

10. Gehörknöchelchenprothese nach einem der vorhergehenden Ansprüche, **dadurch gekennzeichnet, dass** die Prothese oder Teile davon aus einem Material mit Formgedächtnis (=memory effect), insbesondere aus Nitinol hergestellt ist.

11. Gehörknöchelchenprothese nach einem der vorhergehenden Ansprüche, **dadurch gekennzeichnet, dass** die Mittel zur Frequenzanpassung eine Einrichtung zum Verändern der Anlenkungsstelle der Prothese am Ambossfortsatz und/oder am Steigbügel und/oder am Innenohr in Abhängigkeit von einem vorgegebenen Frequenzgang umfassen.

12. Gehörknöchelchenprothese nach einem der vorhergehenden Ansprüche, **dadurch gekennzeichnet, dass** die Massenverteilung der einzelnen Teile der Prothese in Abhängigkeit von einem gewünschten, vorgebbaren Frequenzgang der Schallleitung im Mittelohr berechnet ist.

13. Gehörknöchelchenprothese nach einem der vorhergehenden Ansprüche, **dadurch gekennzeichnet, dass** mindestens eine zusätzliche Masse (23) in Abhängigkeit von einem gewünschten, vorgebbaren Frequenzgang der Schallleitung im Mittelohr vorgesehen ist, die an einem Teil der Gehörknöchelchenkette bzw. der Prothese befestigt werden kann.

14. Gehörknöchelchenprothese nach Anspruch 18, **dadurch gekennzeichnet, dass** die zusätzliche Masse (23) mittels eines zweiten Clips (22) an einem Teil der Gehörknöchelchenkette oder der Prothese befestigt werden kann.

15. Gehörknöchelchenprothese nach einem der vorhergehenden Ansprüche, **dadurch gekennzeichnet, dass** die Prothese mit einem aktiven Vibrationsteil eines aktiven, insbesondere implantierbaren Hörgeräts verbunden ist.

## Claims

1. An auditory ossicle prosthesis (10; 30; 40; 50; 60) which is produced from an elastic material or from a material having at least one articulation, wherein means for tuning are provided for the sound conduction in the middle ear, **characterised in that** the means for tuning comprise lever elements for varying the lever conditions in the chain of auditory ossicles, and **in that** the prosthesis has a least one ball and socket joint.

2. The auditory ossicle prosthesis as set forth in Claim 1, **characterised in that** the prosthesis has a first clip (11; 11') by means of which it can be fastened to the incus extension (20), and **in that** a first rod (12; 12'; 12") is formed on the first clip (11; 11'), which rod terminates in an end designed as a ball (13), which is mounted in a U-shaped socket part (14) which transfers into a second bar (15; 15'; 15") which terminates as a piston (16) or in a further clip.

3. The auditory ossicle prosthesis as set forth in Claim 2, **characterised in that** the U-shaped socket part (14) has, in lateral walls (17), openings (18) in which the ball (13) is mounted.

4. The auditory ossicle prosthesis as set forth in Claim 2 or 3, **characterised in that** the first and/or further clip (11; 11') is formed from two flexible tongues arranged in a V- or U-shape.

5. The auditory ossicle prosthesis as set forth in Claims 2 to 4, **characterised in that** the first and/or further clip (11, 11') is roughened at its contact points with the incus extension (20) and/or the stapes.

6. The auditory ossicle prosthesis as set forth in Claims 2 to 5, **characterised in that** the first and/or further clip (11) has a handle (190.

7. The auditory ossicle prosthesis as set forth in any one of the preceding claims, **characterised in that** the auditory ossicle prosthesis (30; 40; 50) is designed so that can be coupled directly to the inner ear by opening the human cochlea (= cochleotomy) at one end, in particular by means of a piston (16).

8. The auditory ossicle prosthesis as set forth in any one of the preceding claims, **characterised in that** the prosthesis or parts thereof is/are produced from biocompatible plastics, particularly silicone, or from compound fibre materials.

9. The auditory ossicle prosthesis as set forth in any one of the preceding claims, **characterised in that** the prosthesis or parts thereof is/are produced from titanium and/or from gold and/or from tantalum and/or from an alloy of the metals mentioned.

10. The auditory ossicle prosthesis as set forth in any one of the preceding claims, **characterised in that** the prosthesis or parts thereof is/are produced from a material with memory effect, particularly from nitinol.

11. The auditory ossicle prosthesis as set forth in any one of the preceding claims, **characterised in that** the means for tuning comprise a device for varying the point of articulation of the prosthesis to the incus extension and/or to the stapes and/or to the inner ear, as a function of a predetermined frequency response.

12. The auditory ossicle prosthesis as set forth in any one of the preceding claims, **characterised in that** the mass distribution of the individual parts of the prosthesis is calculated as a function of a desired, predeterminable frequency response of the sound conduction in the middle ear.

13. The auditory ossicle prosthesis as set forth in any one of the preceding claims, **characterised in that** at least one additional mass (23) is provided as a function of a desired, predeterminable frequency response of the sound conduction in the middle ear, which mass can be secured to a part of the chain of auditory ossicles or of the prosthesis.

14. The auditory ossicle prosthesis as set forth in Claim 13, **characterised in that** the additional mass (23) can be fastened to a part of the chain of auditory ossicles or of the prosthesis by means of a second clip (22).

15. The auditory ossicle prosthesis as set forth in any one of the preceding claims, **characterised in that** the prosthesis is connected to an active vibration part of an active, in particular implantable, hearing aid.

## Revendications

1. Prothèse d'osselet de l'oreille (10 ; 30 ; 40 ; 50 ; 60), qui est fabriquée à partir d'un matériau élastique ou d'un matériau présentant au moins un assemblage articulé, dans laquelle il est prévu des moyens d'adaptation en fréquence (= syntonisation) pour la conduction acoustique dans l'oreille moyenne, **caractérisée en ce que** les moyens d'adaptation en fréquence comprennent des éléments de levier pour modifier les rapports de levier dans la chaîne des osselets de l'oreille et **en ce que** la prothèse comprend au moins un assemblage articulé à rotule.

2. Prothèse d'osselet de l'oreille selon la revendication 1**, caractérisée en ce que** la prothèse présente une première pince (11 ; 11'), via laquelle elle peut être fixée sur le prolongement d'enclume (20) et **en ce qu'**est formée sur la première pince (11 ; 11') une première tige (12 ; 12' ; 12") qui se termine par une extrémité en forme de bille (13), qui est montée dans une partie de cavité en forme de U (14) qui se fond dans une deuxième tige (15 ; 15' ; 15") qui se termine en forme de piston (16) ou dans une autre pince.

3. Prothèse d'osselet de l'oreille selon la revendication 2, **caractérisée en ce que** la partie de cavité en U (14) présente dans des parois latérales (17) des ouvertures (18), dans lesquelles la bille (13) est montée.

4. Prothèse d'osselet de l'oreille selon la revendication 2 ou 3, **caractérisée en ce que** la première pince et/ou l'autre pince (11 ; 11') est constituée de deux languettes élastiques aménagées en forme de V ou de U.

5. Prothèse d'osselet de l'oreille selon l'une quelconque des revendications 2 à 4, **caractérisée en ce que** la première pince et/ou l'autre pince (11 ; 11') est ou sont rugueuses à ses ou leurs points de contact avec le prolongement d'enclume (20) et/ou avec l'étrier.

6. Prothèse d'osselet de l'oreille selon l'une quelconque des revendications 2 à 5, **caractérisée en ce que** la première pince et/ou l'autre pince (11) présente(nt) une poignée (19).

7. Prothèse d'osselet de l'oreille selon l'une quelconque des revendications précédentes, **caractérisée en ce que** la prothèse (30 ; 40 ; 50) des osselets de l'oreille est conformée de manière à pouvoir être accouplée, au moyen d'une incision de la cochlée humaine (= cochléotomie), à une extrémité, directement à l'oreille interne, en particulier via une queue (16).

8. Prothèse d'osselet de l'oreille selon l'une quelconque des revendications précédentes, **caractérisée en ce que** la prothèse ou des parties de celle-ci est ou sont fabriquées en matériaux synthétiques biocompatibles, en particulier en silicone, ou en matériaux de fibres composites.

9. Prothèse d'osselet de l'oreille selon l'une quelconque des revendications précédentes, **caractérisée en ce que** la prothèse ou des parties de celle-ci sont fabriquées en titane et/ou en or et/ou en tantale et/ou en un alliage des métaux cités.

10. Prothèse d'osselet de l'oreille selon l'une quelconque des revendications précédentes, **caractérisée en ce que** la prothèse ou des parties de celle-ci est ou sont fabriquées en un matériau à mémoire de forme (= memory effect), en particulier en Nitinol.

11. Prothèse d'osselet de l'oreille selon l'une quelconque des revendications précédentes, **caractérisée en ce que** les moyens d'adaptation en fréquence comprennent un dispositif pour modifier le point d'articulation de la prothèse sur le prolongement d'enclume et/ou sur l'étrier et/ou sur l'oreille interne en fonction d'une réponse en fréquence prédéfinie.

12. Prothèse d'osselet de l'oreille selon l'une quelconque des revendications précédentes, **caractérisée en ce que** la distribution de masse des parties individuelles de la prothèse est calculée en fonction d'une réponse en fréquence prédéfinie souhaitée de la conduction acoustique dans l'oreille moyenne.

13. Prothèse d'osselet de l'oreille selon l'une quelconque des revendications précédentes, **caractérisée en ce qu'**au moins une masse supplémentaire (23) est prévue en fonction d'une réponse harmonique prédéfinie souhaitée de la conduction acoustique dans l'oreille moyenne, masse qui peut être fixée sur une partie de la chaîne d'osselets de l'oreille ou de la prothèse.

14. Prothèse d'osselet de l'oreille selon la revendication 13, **caractérisée en ce que** la masse supplémentaire (23) peut être fixée au moyen d'une deuxième pince (22) sur une partie de la chaîne d'osselets de l'oreille ou de la prothèse.

15. Prothèse d'osselet de l'oreille selon l'une quelconque des revendications précédentes, **caractérisée en ce que** la prothèse est raccordée à une partie vibratoire active d'un appareil auditif actif, en particulier implantable.
